(19) Europäisches Patentamt
European Patent Office
Office européen des brevets

(11) Numéro de publication: **0 316 246**
**A1**

(12) # DEMANDE DE BREVET EUROPEEN

(21) Numéro de dépôt: **88420376.1**

(22) Date de dépôt: **09.11.88**

(51) Int. Cl.4: **C 07 D 213/84**
C 07 D 405/12, A 01 N 43/40

(30) Priorité: **12.11.87 FR 8715897**

(43) Date de publication de la demande:
**17.05.89 Bulletin 89/20**

(84) Etats contractants désignés:
**AT BE CH DE ES FR GB GR IT LI LU NL SE**

(71) Demandeur: **RHONE-POULENC AGROCHIMIE**
**14-20, rue Pierre Baizet**
**F-69009 Lyon (FR)**

(72) Inventeur: **Lambert, Claude**
**12, Chemin de Montpellas**
**F-69009 Lyon (FR)**

**Repin, Régis**
**27, Montée Castellane**
**F-69140 Rilleux la Pape (FR)**

**Latorse, Marie-Pascale**
**2ter, Louis Bouquet**
**F-69009 Lyon (FR)**

(74) Mandataire: **Chretien, François et al**
**RHONE-POULENC AGROCHIMIE Service P.I.D. 14-20**
**Rue Pierre Baizet**
**F-69009 Lyon (FR)**

(54) **Nicotinates d'oximes fongicides.**

(57) - Dérivés nicotiniques.
- Ils ont la formule :

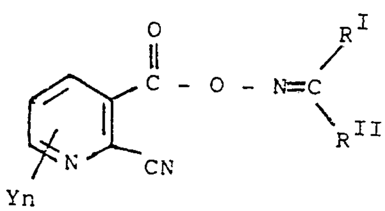

avec $R^1$, $R^2$ identiques ou différents, = H, alcoyle ($C_1$-$C_{18}$) phényle éventuellement substitué, pyridyle ;
Y = H, hal, alcoyle ou alcoxy ou $C_1$-$C_4$ ;
n = entier de 1 à 3.
- Composés fongiques agricoles.

EP 0 316 246 A1

**Description**

La présente invention concerne des compositions fongicides à base de dérivés nicotiniques, des dérivés nicotiniques nouveaux et l'utilisation de dérivés nicotiniques pour la lutte contre les maladies fongiques des plantes.

L'invention a plus particulièrement pour objet des compositions fongicides caractérisées en ce qu'elles contiennent comme matière active au moins un composé de formule :

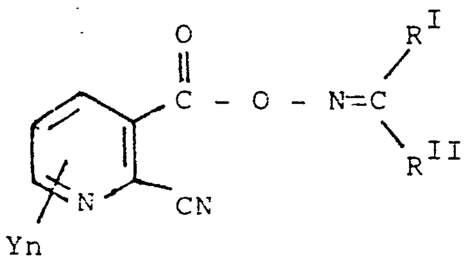

dans laquelle :
-$R^I$ ou $R^{II}$
identiques ou différents sont
. un atome d'hydrogène,
. un radical alcoyle, linéaire ou ramifié, de 1 à 18 atomes de carbone et de préférence de 1 à 4 ;
ou forment, avec l'atome de carbone lié à l'atome d'azote, un cycle à 5 ou 6 chaînons pouvant lui même porter, par l'intermédiaire de deux atomes de carbone contigus, un phénylydène ;
. un radical phényle éventuellement substitué par au moins un substituant choisi dans le groupe comprenant un atome d'halogène, un alcoyle de 1 à 4 atomes de carbone, un alkoxy de 1 à 4 atomes de carbone, un amino éventuellement substitué par au moins un alcoyle de 1 à 4 atomes de carbone, un nitro, encore un alcoylène ($C_1$-$C_3$) dioxy porté par deux atomes de carbone du phényle contigus,
. un radical pyridinyle.
- Y est un atome d'hydrogène ou d'halogène, un radical alcoyle ou alcoxy de 1 à 4 atomes de carbone.
- n est un entier de 1 à 3, ou l'un de ses isomères.

Les composés selon l'invention peuvent être préparés par réaction d'un composé de formule II avec un agent chlorant pour donner un dérivé chlorure d'acide III que l'on fait réagir avec une oxime, selon le schéma (Procédé III B) :

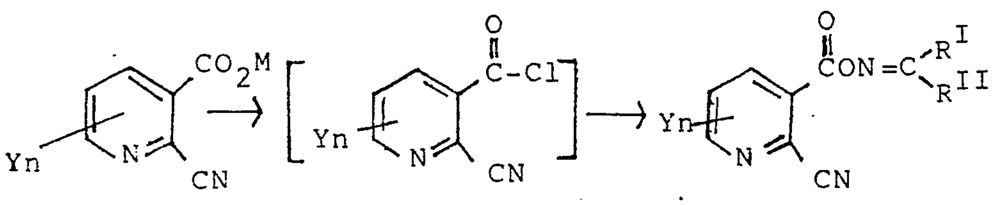

II III I

dans lequel M est un atome de métal alcalin.

Le traitement de II par un agent chlorant, tel que phosgène, chlorure d'oxalyle ou chlorure de thionyle, dans un solvant aliphatique tel qu'un chloroalcane fournit le dérivé III. Celui-ci est transformé en composé I par réaction avec l'oxime appropriée en présence d'une base organique telle que par exemple la pyridine.

Les exemples suivants illustrent la préparation des composés selon l'invention et leurs propriétés fongicides. La structure de ces composés a été vérifiée par spectrographie RMN.

Exemple 1 : 0-[(cyano-2) pyridin-3-yl carbonyl] oxime de l'acétone (composé n° 1)

2,8 g (0,022 mole) de chlorure d'oxalyle sont ajoutés goutte à goutte à une suspension de 4,1 g (0,022 mole) de cyano-2 nicotinate de potassium dans 40 ml de dichlorométhane. Le mélange est ensuite porté au reflux durant une heure. Une solution de 1,46 g (0,02 mole) d'oxime de l'acétone et de 1,6 g (0,02 mole) de pyridine dans 10 ml de dichlorométhane est alors ajoutée au mélange, à 20°C. Le mélange est ensuite agité durant 2

heures à 20° C, lavé à l'eau puis séché sur $Na_2SO_4$. Après évaporation du solvant et cristallisation dans le pentane, on obtient 3,4 g (83 %) d'un solide jaune correspondant à la structure ci-dessus (PF : 92° C).

Exemple 2 : 0[(cyano-2) pyridin-3 yl carbonyl] oxime du chloro-4 benzaldéhyde (composé n° 2)

2,8 g (0,022 mole) de chlorure d'oxalyle sont ajoutés goutte à goutte à une suspension de 4,1 g (0,022 mole) de cyano-2 nicotinate de potassium dans 40 ml de dichlorométhane. Le mélange est ensuite porté au reflux durant une heure. Une solution de 3,1 g (0,02 mole) d'oxime du chloro-4 benzaldéhyde et de 1,6 g (0,02 mole) de pyridine dans 20 ml de dichlorométhane est alors ajoutée au mélange, à 20° C. Le mélange est ensuite agité durant 2 heures à 20° C. Le solvant est évaporé et le résidu traité par 50 ml d'eau. Le solide formé est filtré, lavé à l'eau, puis à l'éther éthylique. On obtient ainsi 4,4 g (77 %) d'un solide blanc correspondant à la structure 2 (PF : 160 °C).

Exemples 3 à 9

En opérant selon l'un des deux exemples précédents, on obtient les composés suivants dont les structures et les caractéristiques physico-chimiques sont rassemblées dans le tableau suivant :

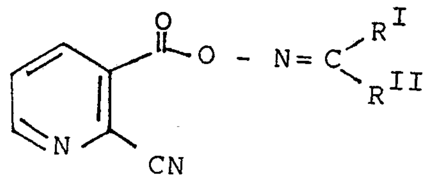

| Composé n° | $R^I$ | $R^{II}$ | Point de fusion (°C) |
|---|---|---|---|
| 1 | $CH_3$ | $CH_3$ | 92 |
| 2 | H | 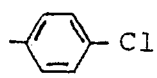 | 160 |
| 3 | H | 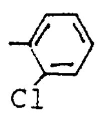 | 159 |
| 4 | H | 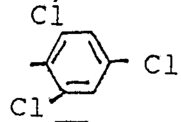 | 161 |
| 5 | H | 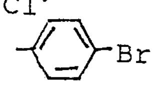 | 93 |
| 6 | H | 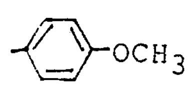 | 134 |
| 7 | H | 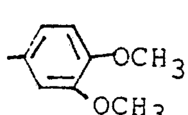 | 152 |
| 8 | H | 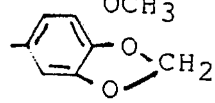 | 142 |
| 9 | H | 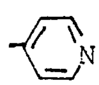 | 157 |

Exemple 10 : Test de serre, sur Piricularia oryzae

Des plants de riz (variété Rosa Marchetti) de hauteur 10 cm groupés en barquettes de 2, sont traités par arrosage au sol, avec une suspension aqueuse contenant :

- 30 mg du produit à tester
- 15 mg d'un agent tensioactif, condensat d'oxyde d'éthylène (20 moles) sur le monoléate de sorbitan
- eau gsp 30 ml

Cette suspension, appliquée à des pots carrés de 7 cm de côté, correspond à une dose d'environ 60 kg/ha de matière active. On laisse absorber le produit par le sol. Une partie des plants de riz n'a pas été traitée pour servir de témoins. Au bout de 24 heures après le traitement on contamine tous les plants de riz avec une suspension de spores de Piricularia oryzae obtenues par grattage d'une culture in vitro, par pulvérisation sur les feuilles à raison de 5 ml de suspension par pot. On laisse incuber les pots pendant 24 h à 25° C sous 100° d'humidité relative .Les plants sont alors transférés en cellule d'observation.

On effectue l'observation au bout de 7 jours après la contamination.

Dans ces conditions on observe qu'à la dose de 60 kg/ha, une inhibition d'au moins 80 % du champignon est obtenue avec les composés 1, 2, 8 et 9.

Cette expérimentation montre bien les propriétés fongicides systémiques des composés selon l'invention et leur action remarquable sur la piriculariose du riz.

Ces compositions peuvent contenir aussi toute sorte d'autres ingrédients tels que, par exemple, des colloïdes protecteurs, des adhésifs, des épaississants, des agents thixotropes, des agents de pénétration, des stabilisants, des séquestrants, etc... ainsi que d'autres matières actives connues à propriétés pesticides (notamment insecticides ou fongicides) ou à propriétés favorisant la croissance des plantes (notamment des engrais) ou à propriétés régulatrices de la croissance des plantes. Plus généralement les composés selon l'invention peuvent être associés à tous les additifs solides ou liquides correspondant aux techniques habituelles de la mise en formulation.

Ces doses d'emploi dans le cas d'une utilisation comme fongicides des composés selon l'invention peuvent varier dans de larges limites, notamment selon la virulence des champignons et les conditions climatiques.

D'une manière générale des compositions contenant 0,5 à 5000 ppm de substance active conviennent bien ; ces valeurs sont indiquées pour les compositions prêtes à l'application. Ppm signifie "partie par million". La zone de 0,5 à 5000 ppm correspond à une zone de $5x10^{-5}$ à 0,5 % (pourcentages pondéraux).

En ce qui concerne les compositions adaptées au stockage et au transport, elles contiennent plus avantageusement de 0,5 à 95 % (en poids) de substance active.

Ainsi donc, les compositions à usage agricole selon l'invention peuvent contenir les matières actives selon l'invention dans de très larges limites, allant de $5.10^{-5}$% à 95 % (en poids).

Selon ce qui a déjà été dit les composés selon l'invention sont généralement associés à des supports et éventuellement des agents tensioactifs.

Par le terme "support", dans le présent exposé, on désigne une matière organique ou minérale, naturelle ou synthétique, avec laquelle la matière active est associée pour faciliter son application sur la plante, sur des graines ou sur le sol. Ce support est donc généralement inerte et il doit être acceptable en agriculture, notamment sur la plante traitée. Le support peut être solide (argiles, silicates naturels ou synthétiques, silice,résines, cires, engrais solides, etc...) ou liquide (eau, alcools, cétones, fractions de pétrole, hydrocarbures aromatiques ou paraffiniques, hydrocarbures chlorés, gaz liquéfiés, etc...).

L'agent tensioactif peut être un agent émulsionnant, dispersant ou mouillant de type ionique ou non ionique. On peut citer par exemple des sels d'acides polyacryliques, des sels d'acides lignosulfoniques, des sels d'acides phénolsulfoniques ou naphtalènesulfoniques, des polycondensats d'oxyde d'éthylène sur des alcools gras ou sur des acides gras ou sur des amines grasses, des phénols substitués (notamment des alkylphénols ou des arylphénols), des sels d'esters d'acides sulfosucciniques, des dérivés de la taurine (notamment des alkyltaurates), des esters phosphoriques d'alcools ou de phénols polyoxyéthylés. La présence d'au moins un agent tensioactif est généralement indispensable lorsque la matière active et/ou le support inerte ne sont pas solubles dans l'eau et que l'agent vecteur de l'application est l'eau.

Les compositions utilisées dans l'invention peuvent être sous des formes assez diverses, solides ou liquides.

Comme formes de compositions solides, on peut citer les poudres pour poudrage (à teneur en matières actives pouvant aller jusqu'à 100 %) et les granulés, notamment ceux obtenus par extrusion, par compactage, par imprégnation d'un support granulé, par granulation à partir d'une poudre (la teneur en composé de formule (I) dans ces granulés étant entre 0,5 et 80 % pour ces derniers cas).

Comme formes de compositions liquides ou destinées constituer des compositions liquides lors de l'application, on peut citer les solutions, en particulier les concentrés solubles dans l'eau, les concentrés émulsionnables, les émulsions, les suspensions concentrées, les aérosols, les poudres mouillables (ou poudre à pulvériser), les granulés, les pâtes.

Les concentrés émulsionnables ou solubles comprennent le plus souvent 10 à 80 % de matière active, les émulsions ou solutions prêtes à l'application contenant, quant à elles, 0,001 à 20 % de matière active. En plus de la matière active et du solvant, les concentrés émulsionnables peuvent contenir, quand c'est nécessaire, un co-solvant approprié et de 2 à 20 % d;additifs appropriés, comme des stabilisants, des agents tensioactifs,

notamment des émulsifs, des agents de pénétration, des inhibiteurs de corrosion, des colorants, des adhésifs.

A partir de ces concentrés, on peut obtenir par dilution avec de l'eau des émulsions de toute concentration désirée, qui conviennent particulièrement à l'application sur les cultures.

Les suspensions concentrées, également applicables en pulvérisation, sont préparées de manière à obtenir un produit fluide stable ne se déposant pas et elles contiennent habituellement de 10 à 75 % de matière active, de 0,5 à 15 % d'agents tensioactifs, de 0,1 à 10 % d'agents thixotropes, de 0 à 10 % d'additifs appropriés, comme des anti-mousses, des inhibiteurs de corrosion, des stabilisants, des agents de pénétration et des adhésifs et, comme support, de l'eau ou un liquide organique dans lequel la matière active est peu ou pas soluble : certaines matières solides organiques ou des sels minéraux peuvent être dissous dans le support pour aider à empêcher la sédimentation ou comme antigels pour l'eau.

A titre d'exemple, voici la composition de plusieurs suspensions aqueuses selon l'invention :

Exemple SA1 :

On prépare une suspension aqueuse comprenant :

| | |
|---|---|
| - matière active (composé n° 4).... | 500 g/l |
| - agent mouillant (alcool synthétique en $C_{13}$ polyéthoxylé) .... | 10 g/l |
| - agent dispersant (phosphate de polyaryl phénol éthoxylé salifié) .... | 50 g/l |
| - antigel (propylèneglycol) .... | 100 g/l |
| - épaissisant (polysacharide) .... | 1,6 g/l |
| - biocide (méthylhydroxy-4 benzoate sodé) .... | 3,3 g/l |
| - eau .... | Q.S.P.1 litre. |

On obtient ainsi une suspension concentrée fluide.

Exemple SA2 - Suspension aqueuse

On prépare une suspension aqueuse comprenant :

| | |
|---|---|
| - matière active (composé n° 7).... | 100 g/l |
| - agent mouillant (alkylphénol polyéthoxylé .. | 5 g/l |
| - agent dispersant (Naphtalène sulfonate de Na | 10 g/l |
| - antigel (Propylèneglycol) .... | 100 g/l |
| - épaissisant (Polysacharide) .... | 3 g/l |
| - biocide (Formaldéhyde) .... | 1 g/l |
| - eau .... | Q.S.P.1 litre. |

Exemple SA3 - Suspension aqueuse :

On prépare une suspension aqueuse comprenant :

| | |
|---|---|
| - matière active (composé n° 9).... | 250 g/l |
| - agent mouillant (alcool synthétique en $C_{13}$ polyéthoxylé .... | 10 g/l |
| - agent dispersant (lignosulfonate de solium) | 15 g/l |
| - antigel (urée) .... | 50 g/l |
| - épaississant (Polysacharide) .... | 2,5 g/l |
| - biocide (Formaldéhyde) .... | 1 g/l |
| - eau .... | Q.S.P.1 litre. |

Les poudres mouillables (ou poudre à pulvériser) sont habituellement préparées de manière qu'elles contiennent 10 à 95 % de matière active, et elles contiennent habituellement, en plus du support solide, de 0 à 5 % d'un agent mouillant, de 3 à 10 % d'un agent dispersant, et, quand c'est nécessaire, de 0 à 10 % d'un ou plusieurs stabilisants et/ou autres additifs, comme des agents de pénétration, des adhésifs, ou des agents antimottants, colorants, etc...

A titre d'exemple, voici la composition de plusieurs poudres mouillables.

Exemple PM1 : Poudre mouillable à 10 %

| | |
|---|---|
| - matière active (composé n° 4).... | 10 % |
| - alcool synthétique oxo de type ramifié, en $C_{13}$ éthoxylé par 8 à 10 oxyde d'éthylène (agent mouillant) .... | 0,75 % |
| - lignosulfonate de calcium neutre (agent dispersant .... | 12 % |
| - carbonate de calcium (charge inerte) .... | qsp 100 % |

Exemple PM2 : Poudre mouillable à 75 % contenant les mêmes ingrédients que dans l'exemple précédent, dans les proportions ci-après :

| | |
|---|---|
| - matière active (composé n° 7) .... | 75 % |
| - agent mouillant .... | 1,50 % |
| - agent dispersant .... | 8 % |
| - carbonate de calcium (charge inerte) .... | qsp 100 % |

Exemple PM3 : Poudre mouillable à 90 %

| | |
|---|---|
| - matière active (composé n° 9) .... | 90 % |
| - alcool gras éthoxylé (agent mouillant .... | 4 % |
| - styrylphénol éthoxylé (agent dispersant) | 6 % |

Exemple PM4 ; Poudre mouillable à 50 %

| | |
|---|---|
| - matière active (composé n° 4 selon l'invention).. | 50 % |
| - mélange de tensio-actifs anioniques et non ioniques (agent mouillant) .... | 2,5 % |
| - lignosulfonate de sodium neutre (agent dispersant) .... | 5 % |
| - argile kaolinique (support inerte) .... | 42,5 % |

Pour obtenir ces poudres à pulvériser ou poudres mouillables, on mélange intimement la matière active dans des mélangeurs appropriés avec les substances additionnelles et on broie avec des moulins ou autres broyeurs appropriés. On obtient par là des poudres à pulvériser dont la mouillabilité et la mise en suspension sont avantageuses ; on peut les mettre en suspension avec de l'eau à toute concentration désirée et cette suspension est utilisable très avantageusement en particulier pour l'application sur les feuilles des végétaux.

Les composés de formule (I) peuvent encore être utilisés sous forme de poudres pour poudrage ; on peut aussi utiliser une composition comprenant 50 g de matière active et 950 g de talc; on peut aussi utiliser une composition comprenant 20 g de matière active, 10 g de silice finement divisée et 970 g de talc ; on mélange et broie ces constituants et on applique le mélange par poudrage.

Les granulés pour épandage ont des dimensions comprises entre 0,1 et 2 mm et peuvent être fabriqués par agglomération ou imprégnation. En général, les granulés contiennent 0,5 à 25 % de matière active et 0 à 10 % d'additifs comme des stabilisants, des agents de modification à libération lente, des liants et des solvants.

Voici deux exemples de composition de granulé :

Exemples G 1 et G 2 :

| | | |
|---|---|---|
| - matière active (composé n° 2) .... | 50 g | 200 g |
| - propylène glycol.... | 50 g | 50 g |
| - éther de cétyle et de polyglycol.... | 2,5 g | 2,5 g |
| - polyéthylène glycol.... | 35 g | 35 g |
| - kaolin (granulométrie : 0,3 à 0,8 mm). | 910 g | 760 g |

Les composés selon l'invention peuvent encore être formulés sous forme de solutions organiques encapsulées, notamment par polymérisation interfaciale, dans des capsules à paroi polymériques, par exemple à base de polyamides de polyurées ou de polyamide urées. Ces capsules se trouvent à l'état de dispersion aqueuse concentrées que l'on peut diluer au moment de l'emploi pour obtenir une bouillie de pulvérisation.

Les composés selon l'invention peuvent être avantageusement formulés sous la forme de granulés dispersibles dans l'eau également compris dans le cadre de l'invention.

Ces granulés dispersibles, de densité apparente généralement comprise entre environ 0,3 et 0,6, ont une dimension de particules généralement comprise entre environ 150 et 2000 et de préférence entre 300 et 1500 microns.

La teneur en matière active (composé n° 4) de ces granulés est généralement comprise entre environ 1 % et 90 %, et de préférence entre 25 % et 90 %.

Le reste du granulé est essentiellement composé d'une charge solide et éventuellement d'adjuvants tensio-actifs conférant au granulé des propriétés de dispersibilité dans l'eau. Ces granulés peuvent être

essentiellement de deux types distincts selon que la charge retenue est soluble ou non dans l'eau. Lorsque la charge est hydrosoluble, elle peut être minérale et de préférence organique. On a obtenu d'excellents résultats avec l'urée. Dans le cas d'une charge insoluble, celle-ci est de préférence minérale, comme par exemple le kaolin ou la bentonite. Elle est alors accompagnée d'agents tensio-actifs (à raison de 2 à 20 % en poids du granulé), adjuvants tensio-actifs dont plus de la moitié est constituée par au moins un agent dispersant, essentiellement anionique, tel qu'un poly(naphtalène sulfonate alcalin ou alcalino terreux) ou un lignosulfonate alcalin ou alcalino-terreux, le reste étant constitué par des mouillants non ioniques ou anioniques tel qu'un alcoyl naphtalène sulfonate alcalin ou alcalino-terreux.

Par ailleurs, bien que cela ne soit pas indispensable, on peut ajouter d'autres adjuvants tels que des agents anti-mousse.

Le granulé selon l'invention peut être préparé par mélange des ingrédients nécessaires puis granulation selon plusieurs techniques en soi connues (drageoir, lit fluide, atomiseur, extrusion, etc..). On termine généralement par un concassage suivi d'un tamisage à la dimension de particule choisie dans les limites mentionnées ci-dessus.

De préférence, il est obtenu par extrusion, en opérant comme indiqué dans les exemples ci-après.

Exemple GD1 : Granulés dispersibles à 90 % de matière active

Dans un mélangeur, on mélange 90 % en poids de matière active (composé n° 7) et 10 % d'urée en perles. Le mélange est ensuite broyé dans un broyeur à broches. On obtient une poudre que l'on humidifie avec environ 8 % en poids d'eau. La poudre humide est extrudée dans une extrudeuse à rouleau perforé. On obtient un granulé qui est séché, puis concassé et tamisé, de façon à ne garder respectivement que les granulés d'une dimension comprise entre 150 et 2000 microns.

Exemple GD2 : Granulés dispersibles à 75 % de matière active

Dans un mélangeur, on mélange les constituants suivants :

| | |
|---|---|
| - matière active (composé n° 9) .... | 75 % |
| - agent mouillant (alkylnaphtalène sulfonate de sodium .... | 2 % |
| - agent dispersant (polynaphtalène sulfonate de sodium) .... | 8 % |
| - charge inerte insoluble dans l'eau (kaolin) .... | 15 % |

Ce mélange est granulé en lit fluide, en présence d'eau, puis séché, concassé et tamisé de manière à obtenir des granulés de dimension comprise entre 0,16 et 0,40 mm.

Ces granulés peuvent être utilisés seuls, en solution ou dispersion dans de l'eau de manière à obtenir la dose cherchée. Ils peuvent aussi être utilisés pour préparer des associations avec d'autres matières actives, notamment fongicides, des dernières étant sous la forme de poudres mouillables, ou de granulés ou suspensions aqueuses.

Comme cela a déjà été dit, les dispersions et émulsions aqueuses, par exemple des compositions obtenues en diluant à l'aide d'eau une poudre mouillable ou un concentré émulsionnable selon l'invention, sont comprises dans le cadre général des compositions utilisables dans la présente invention. Les émulsions peuvent être du type eau-dans-l'huile ou huile-dans-l'eau et elles peuvent avoir une consistance épaisse comme celle d'une "mayonnaise".

L'invention concerne de plus un procédé de traitement des végétaux et notamment le riz et autres céréales contre les maladies causées par les champignons phytopathogènes appartenant aux groupes les plus variées et notamment sur Pyricularia oryzae sensibles ou résistants à divers fongicides notamment à la kasugamycine et à l'iprobenfos.

Ce procédé est caractérisé en ce qu'il consiste à appliquer sur ces végétaux ou leur lieu de culture une quantité efficace d'une composition contenant comme matière active un composé selon la formule (I). Par "quantité efficace" on entend une quantité suffisante pour permettre le contrôle et la destruction des champignons présents sur ces végétaux. Les doses d'utilisation peuvent toutefois varier dans de larges limites selon le champignon à combattre, le type de culture, les conditions climatiques, et selon le composé utilisé.

Par végétaux on entend des plantes ou parties de plantes (feuillage, racines, semences etc...).

En pratique des doses allant de 1 g/hl à 500 g/hl correspondant sensiblement à des doses de matière active par hectare de 10 g/ha à 20 000 g/ha environ donnent généralement de bons résultats.

**Revendications**

1) Dérivés de formule :

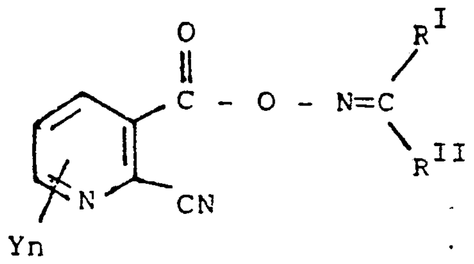

dans laquelle :
-$R^{I}$ ou $R^{II}$
identiques ou différents sont
. un atome d'hydrogène,
. un radical alcoyle, linéaire ou ramifié, de 1 à 18 atomes de carbone ;
ou forment, avec l'atome de carbone lié à l'atome d'azote, un cycle à 5 ou 6 chaînons pouvant lui même porter, par l'intermédiaire de deux atomes de carbone contigus, un phénylydène ;
. un radical phényle éventuellement substitué par au moins un substituant choisi dans le groupe comprenant un atome d'halogène, un alcoyle de 1 à 4 atomes de carbone, un alkoxy de 1 à 4 atomes de carbone, un amino éventuellement substitué par au moins un alcoyle de 1 à 4 atomes de carbone, un nitro, encore un alcoylène ($C_1$-$C_3$) dioxy porté par deux atomes de carbone du phényle contigus,
. un radical pyridinyle.
- Y est un atome d'hydrogène ou d'halogène, un radical alcoyle ou alcoxy de 1 à 4 atomes de carbone.
- n est un entier de 1 à 3, ou l'un de ses isomères.

2) Dérivés selon la revendication 1 caractérisés en ce que dans leur fomule $R^1$ et $R^2$ identiques ou différents sont un atome d'hydrogène, un alcoyle de 1 à 4 atomes de carbone ou un phényle éventuellement substitué.

3) Procédé de préparation des dérivés selon l'une des revendications 1 et 2 caractérisé en ce qu'on effectue la réaction d'un composé de formule II avec un agent chlorant pour donner un dérivé chlorure d'acide III que l'on fait réagir avec une oxime, selon le schéma (Procédé III B) :

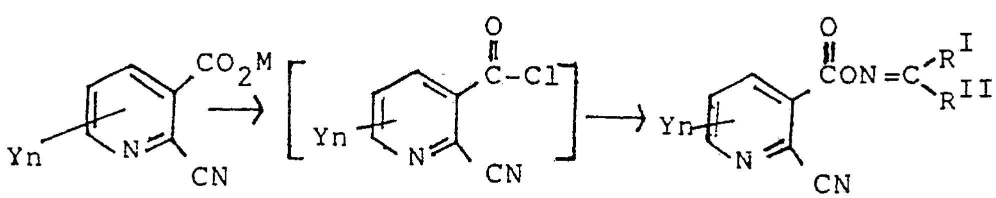

II III I

dans lequel M est un atome de métal alcalin, avec d'abord le traitement de II par un agent chlorant, tel que phosgène, chlorure d'oxalyle ou chlorure de thionyle, dans un solvant aliphatique tel qu'un chloroalcane fournit le dérivé III puis la transformation de celui-ci en composé I par réaction avec l'oxime appropriée en présence d'une base organique.

4) Compositions fongicides caractérisées en ce qu'elles contiennent comme matière active au moins un composé selon l'une des revendications 1 et 2.

5) Procédé de lutte contre les maladies fongiques des plantes caractérisé en ce que on applique à celles-ci ou à leur lieu de culture au moins un composé selon l'une des revendications 1 et 2.

Office européen des brevets

# RAPPORT DE RECHERCHE EUROPEENNE

Numero de la demande

EP 88 42 0376

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl.4) |
|---|---|---|---|
| A | US-A-4 244 959 (CHEVRON RESEARCH)<br>* Revendications *<br>----- | 1,4 | C 07 D 213/84<br>C 07 D 405/12<br>A 01 N 43/40 |

| DOMAINES TECHNIQUES RECHERCHES (Int. Cl.4) |
|---|
| C 07 D 213/00<br>C 07 D 405/00 |

Le présent rapport a été établi pour toutes les revendications

| Lieu de la recherche | Date d'achèvement de la recherche | Examinateur |
|---|---|---|
| LA HAYE | 13-02-1989 | VAN BIJLEN H. |

**CATEGORIE DES DOCUMENTS CITES**

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons

& : membre de la même famille, document correspondant

EPO FORM 1503 03.82 (P0402)